# EUROPEAN PATENT SPECIFICATION

(11) **EP 3 986 387 B1**
(45) Date of publication and mention of the grant of the patent: **11.02.2026**
(21) Application number: 20827376.3
(22) Date of filing: 16.06.2020
(51) Int. Cl.: A61K 31/05, A61K 31/201, A61K 31/202, A61K 31/352, A61K 36/02, A61K 9/107, A23L 33/12, A61P 3/04, A61P 25/00, A61P 25/20, A61P 25/22, A61P 25/24

(54) **COMPOSITION COMPRISING NANOCHLOROPSIS OIL AND CANNABIDIOL AND MEDICAL USES THEREOF**
ZUSAMMENSETZUNGEN, DIE NANOCHLOROPSIS-ÖL UND CANNABIDIOL ENTHALTEN, UND IHRE MEDIZINISCHE VERWENDUNG
COMPOSITIONS COMPENANT DE L'HUILE DE NANOCHLOROPSIS ET DU CANNABIDIOL ET LEURS UTILISATIONS MÉDICALES

(30) Priority: 18.06.2019 US 201962862708 P
(43) Date of publication of application: 27.04.2022
(73) Proprietor: Vaxa Technologies Ltd., 1200000 Rosh (IL)
(72) Inventor: BASHAN, Ohad, 44935 Sde Varburg (IL); BERZIN, Isaac, 9350201 Jerusalem (IL)
(74) Representative: Pearl Cohen Zedek Latzer Baratz UK LLP
(86) International application number: PCT/IL2020/050663
(87) International publication number: WO 2020/255123

(56) References cited:
- WO-A1-2014/105576
- WO-A1-2015/196250
- WO-A1-2016/144376
- WO-A1-2017/009711
- WO-A1-2018/145213
- WO-A1-2019/070885
- US-A1- 2016 081 927
- US-A1- 2019 000 795
- SCHULTZ HANK: "Neptune to partner in researching krill oil as CBD carrier bioavailability enhancer", NUTRAINGREDIENTS USA, 23 January 2018 (2018-01-23), pages 1 - 3, XP093051944, Retrieved from the Internet <URL:https://www.nutraingredients-usa.com/Article/2018/01/23/Neptune-to-partner-in-researching-krill-oil-as-CBD-carrier-bioavailability-enhancer> [retrieved on 20230605]
- MICHAEL L KAGAN ET AL: "Acute appearance of fatty acids in human plasma - a comparative study between polar-lipid rich oil from the microalgae Nannochloropsis oculata and krill oil in healthy young m", LIPIDS IN HEALTH AND DISEASE, BIOMED CENTRAL, LONDON, GB, vol. 12, no. 1, 15 July 2013 (2013-07-15), pages 102, XP021156751, ISSN: 1476-511X, DOI: 10.1186/1476-511X-12-102
- L. B. BLANCHARD: "Insomnia and exacerbation of anxiety associated with high-EPA fish oil supplements after successful treatment of depression", OXFORD MEDICAL CASE REPORTS, vol. 2015, no. 3, 24 March 2015 (2015-03-24), pages 244 - 245, XP093156672, ISSN: 2053-8855, Retrieved from the Internet <URL:https://www.ncbi.nlm.nih.gov/pmc/articles/PMC4664844/pdf/omv024.pdf> DOI: 10.1093/omcr/omv024
- KIMBERLY A. BABSON: "Cannabis, Cannabinoids, and Sleep: a Review of the Literature", CURRENT PSYCHIATRY REPORTS, vol. 19, no. 4, 27 March 2017 (2017-03-27), US, XP093156671, ISSN: 1523-3812, Retrieved from the Internet <URL:http://link.springer.com/article/10.1007/s11920-017-0775-9/fulltext.html> DOI: 10.1007/s11920-017-0775-9
- MA XIAO-NIAN ET AL: "Lipid Production from Nannochloropsis", MARINE DRUGS, vol. 14, no. 4, 25 March 2016 (2016-03-25), pages 61, XP055808900, DOI: 10.3390/md14040061
- ANONYMOUS: "EPA-rich Nannochloropsis oculata oil", COMPOSITIONAL GUIDELINE, 4 August 2022 (2022-08-04), pages 1 - 3, XP093156612, Retrieved from the Internet <URL:https://www.tga.gov.au/resources/resource/compositional-guidelines/epa-rich-nannochloropsis-oculata-oil>

## Description

### FIELD OF THE INVENTION

The present disclosure relates to compositions comprising lipids. More particularly, the present disclosure relates to compositions comprising polar lipids and Cannabidiol, and uses thereof. The present invention relates to compositions comprising Nannochloropsis oil and Cannabidiol "CBD", and uses thereof, as defined in the claims.

### BACKGROUND OF THE INVENTION

As the primary homeostatic regulator of human physiology, the human endocannabinoid system (ECS) plays a major role in the sleep-wake cycle and other circadian processes. The endocannabinoid system comprises cannabinoid receptors, their endogenous ligands, the endocannabinoids, and their biosynthetic and degradation enzymes.

How humans fall asleep, stay asleep, wake up, and remain awake is part of an internal biological process regulated by the circadian rhythms and the endocannabinoid system. The circadian rhythms govern a diverse array of actions in the body, including hormone production, heart rate, metabolism, and when to go to sleep and wake up.

Evidence of a strong relationship between endocannabinoid system and the circadian rhythms was observed in the sleep-wake cycle fluctuations of anandamide and 2-AG (the brain's own marijuana-like molecules), along with the metabolic enzymes that create and break down these endogenous cannabinoid compounds.

Anandamide is present in the brain at higher levels at night and works with the endogenous neurotransmitters oleamide and adenosine to generate sleep. Conversely, 2AG is higher during the day, suggesting that it is involved in promoting wakefulness.

The highly complex sleep-wake cycle is driven by a variety of neurochemicals and molecular pathways. Both anandamide and 2AG activate CB1 cannabinoid receptors that are concentrated in the central nervous system, including parts of the brain associated with regulating sleep.

CB1 receptors modulate neurotransmitter release in a manner that dials back excessive neuronal activity, thereby reducing anxiety, pain, and inflammation. CB1 receptor expression is thus a key factor in modulating sleep homeostasis. This is not the case, however, with respect to the CB2, the cannabinoid receptor located primarily in immune cells, the peripheral nervous system, and metabolic tissue. Whereas CB 1 receptor expression reflects cyclical circadian rhythms, no such fluctuations have been described for the CB2 receptor.

Cannabidiol (CBD) is one of many cannabinoid compounds found in cannabis. It does not appear to alter consciousness or trigger a "high." A recent surge in scientific publications has found preclinical and clinical evidence documenting value for CBD in some neuropsychiatric disorders, including epilepsy, anxiety, and schizophrenia. Evidence points toward a calming effect for CBD in the central nervous system. Interest in CBD as a treatment of a wide range of disorders has exploded, yet few clinical studies of CBD exist in the psychiatric literature.

Administration of CBD has been shown to have differential effects on sleep based on dose. In a study among individuals with insomnia, results suggested that administration of high-dose (160 mg/day) of CBD increased total sleep time and decreased the frequency of arousals during the night while low-dose CBD has been associated with increased wakefulness.

Nevertheless, the higher doses of CBD that studies suggest are therapeutic for anxiety and insomnia, may also increase mental sedation In addition, the current retail cost of CBD could make the use high-dose administration of CBD cost prohibitive.

Schultz discloses the use of krill oil as CBD carrier ("Neptune to partner in researching krill oil as CBD carrier bioavailability enhancer", NutraIngredients USA, 23 January 2018, pages 1-3, URL:https://www.nutraingredients-usa.com/Article/2018/01/23/Neptune-to-partner-in-researching-krill-oil-as-CBD-carrier-bioavailability-enhancer).

Babson discloses a review on cannabis, cannabinoids and sleep ("Cannabis, Cannabinoids, and Sleep: a Review of the Literature", CURRENT PSYCHIATRY REPORTS, vol. 19, no. 4, 27 March 2017, DOI: 10.1007/s11920-017-0775-9, URL: http://link.springer.com/article/10.1007/s11920-017-0775-9/fulltext.html).

WO 2017/009711 discloses phospholipid preparations for the improvement of sleep and/or treatment of sleep disorders, methods of improving sleep and/or treating sleep disorders comprising administering the same.

### SUMMARY OF THE INVENTION

The present invention provides a composition comprising: Nannochloropsis oil and Cannabidiol "CBD".

In some embodiments, the CBD is extracted from a plant source. In other embodiments, said CBD is synthetic or semi-synthetic.

In some embodiments, the Nannochloropsis oil forms at least 20%wt of the composition and/or the CBD forms at least 10%wt of the composition. In further embodiments, the ratio between the Nannochloropsis oil and CBD is 1:1.

In an embodiment, the Nannochloropsis oil includes glycolipid and phospholipid Eicosapentaenoic acid "EPA".

In some embodiments, the composition is in the form of an oily solution. In some embodiments, the composition is in the form of an oil-in-water solution. In some embodiments, the composition is in the form of a dry powder.

The compositions of the present invention may be provided in admixture with pharmaceutically acceptable auxiliaries, and optionally other therapeutic agents. The auxiliaries may be *"acceptable"* in the sense of being compatible with the other ingredients of the composition and not deleterious to the recipients thereof.

Pharmaceutical compositions may include those suitable for oral, rectal, nasal, topical (including transdermal, buccal and sublingual), vaginal or parenteral (including subcutaneous, intramuscular, intravenous and intradermal) administration or administration via an implant. The compositions may be prepared by any method well known in the art of pharmacy.

Such methods may include the step of bringing in association compounds of the invention or combinations thereof with any auxiliary agent. The auxiliary agent(s), also named accessory ingredient(s), may include those conventional in the art, such as carriers, fillers, binders, diluents, disintegrants, lubricants, colorants, flavouring agents, anti-oxidants, and wetting agents.

Pharmaceutical compositions suitable for oral administration may be presented as discrete dosage units such as pills, tablets, dragées or capsules, or as a powder or granules, or as a solution or suspension. The active ingredient may also be presented as a bolus or paste. The compositions can further be processed into a suppository or enema for rectal administration.

The pharmaceutical composition, as hereinbefore described, may be provided in combination with packaging material, including instructions for the use of the composition for a use as hereinbefore described.

For parenteral administration, suitable compositions may include aqueous and nonaqueous sterile injection. The compositions may be presented in unit-dose or multi-dose containers, for example sealed vials and ampoules, and may be stored in a freeze-dried (lyophilised) condition requiring only the addition of sterile liquid carrier, for example water, prior to use. For transdermal administration, e.g. gels, patches or sprays may be contemplated. Compositions or formulations suitable for pulmonary administration e.g. by nasal inhalation include fine dusts or mists which may be generated by means of metered dose pressurized aerosols, nebulisers or insufflators.

The exact dose and regimen of administration of the composition may necessarily be dependent upon the therapeutic or nutritional effect to be achieved and may vary with the particular formula, the route of administration, and the age and condition of the individual subject to whom the composition is to be administered.

The present invention provides a composition of the invention for use as a medicine. The present invention provides a composition of the present invention, for use in the treatment of a disease or disorder selected from sleep disorder, insomnia and any combinations thereof.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The present invention provides a composition comprising: Nannochloropsis oil and CBD. The present invention also provides a composition including Nannochloropsis oil and CBD, for use as a medicine. In some embodiments, the composition of the present invention is useful in treating insomnia.

As used herein, to a ***"polar lipid comprising at least one PUFA moiety"*** may encompass a lipid having a glycerol or a sphingosine backbone substituted at least one moiety of poly-unsaturated fatty acids (which may be the same or different) and at least one polar group being either a phosphatidyl moiety (such as for example phosphatidylcholine, phosphatidylethanolamine phosphatidylserine, phosphatidylinositols, phosphatidic acids and so forth) or a monosaccharide or oligosaccharide moiety.

The *PUFA moiety* may refer to any carboxylic acid moiety having an aliphatic chain of between about 4 to 28 carbon atoms and at least one double bond between at least two carbon atoms. Said at least one double bond can be in a cis, trans, E or Z configuration.

A polar lipid source may be a plant source. The plant source may be a microalgae. A microalgae may be selected from *Nannochloropsis, Isochrysis, Chlorella* and any combinations thereof.

When referring to CBD it should be understood to encompass a compound of formula (1), including any enantiomer, or diastereomer.

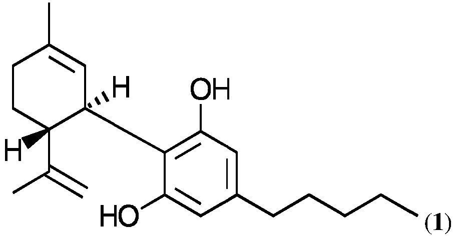

In some embodiments, the CBD is extracted from a plant source. In other embodiments, said CBD is synthetic or semi-synthetic.

In some embodiments, the Nannochloropsis oil forms at least 20%wt of the composition. In other embodiments, the CBD forms at least 10%wt of the composition. In further embodiments, the ratio between the Nannochloropsis oil and CBD is 1:1.

In some embodiments, the EPA is at least 20% of said composition. In other embodiments, said CBD is at least 10% of said composition.

In some embodiments, a composition of the invention is in the form of an oily solution. In some embodiments, a composition of the invention is in the form of an oil-in-water solution. In some embodiments, a composition of the invention is in the form of a dry powder.

The compositions of the present invention may be provided in admixture with pharmaceutically acceptable auxiliaries, and optionally other therapeutic agents. The auxiliaries may be *"acceptable"* in the sense of being compatible with the other ingredients of the composition and not deleterious to the recipients thereof.

Pharmaceutical compositions may include those suitable for oral, rectal, nasal, topical (including transdermal, buccal and sublingual), vaginal or parenteral (including subcutaneous, intramuscular, intravenous and intradermal) administration or administration via an implant. The compositions may be prepared by any method well known in the art of pharmacy.

Such methods may include the step of bringing in association compounds used in the invention or combinations thereof with any auxiliary agent. The auxiliary agent(s), also named accessory ingredient(s), may include those conventional in the art, such as carriers, fillers, binders, diluents, disintegrants, lubricants, colorants, flavouring agents, anti-oxidants, and wetting agents.

Pharmaceutical compositions suitable for oral administration may be presented as discrete dosage units such as pills, tablets, dragées or capsules, or as a powder or granules, or as a solution or suspension. The active ingredient may also be presented as a bolus or paste. The compositions can further be processed into a suppository or enema for rectal administration.

The composition of the present invention may be provided in combination with packaging material, including instructions for the use of the composition for a use as hereinbefore described.

For parenteral administration, suitable compositions may include aqueous and nonaqueous sterile injection. The compositions may be presented in unit-dose or multi-dose containers, for example sealed vials and ampoules, and may be stored in a freeze-dried (lyophilised) condition requiring only the addition of sterile liquid carrier, for example water, prior to use. For transdermal administration, e.g. gels, patches or sprays may be contemplated. Compositions or formulations suitable for pulmonary administration e.g. by nasal inhalation include fine dusts or mists which may be generated by means of metered dose pressurized aerosols, nebulisers or insufflators.

The exact dose and regimen of administration of the composition may necessarily be dependent upon the therapeutic or nutritional effect to be achieved and may vary with the particular formula, the route of administration, and the age and condition of the individual subject to whom the composition is to be administered.

The present invention further provides a composition comprising Nannochloropsis oil and CBD, for use in the treatment of a disease or disorder selected from sleep disorder, insomnia, and any combinations thereof.

Some experiments showing the effect of the combination of CBD and four different omega-3 compounds (Fish Oil (Non-polar DHA+EPA), Fish oil (Non-polar EPA), Schizochytrium Oil (Non-Polar DHA), Nannochloropsis Oil (polar EPA)) were conducted. Daily dosage of omega 3 (DHA and/or EPA) was 500 ml.

***Materials:*** CBD: Fish oil (non-polar DHA+EPA), Schizochytrium Oil (non-polar DHA), Nannochloropsis Oil (polar-EPA), Fish oil (non-polar EPA).

***Experimental Design:*** Sleep quality was measured in this study, as an efficacy indication. The experiment included 6 groups (3 participants per group) 8 women and 10 men, with similar Pittsburg Sleep Quality Index (PSQI) rating (11-13), indicating poor sleeping quality. The average age for the participants was 37.2. All 18 participants completed sleep assessments at the onset of CBD treatment and at the monthly follow-ups.

All participants were given CBD and/or Omega 3 every evening, after dinner, per table 1 below. Sleep quality was tracked on a monthly basis, for a period of up to 4 months, using the PSQI. This Index is a self-report measure that assesses the quality of sleep during a 1-month period. It consists of 19 items that have been found to be reliable and valid in the assessment of a range of sleep-related problems. The PSQI measures several different aspects of sleep, offering seven component scores and one composite score. The component

**Table 1:**

| | **No CBD** | | | | | | **With CBD** | | | | | |
|---|---|---|---|---|---|---|---|---|---|---|---|---|
| | **PSQI** | | **PSQI** | | **PSQI** | | **PSQI** | | **PSQI** | | **ΔPSQI** | **Sedation** |
| | **M0** | **STDV** | **M1** | **STDV** | **M2** | **STDV** | **M3** | **STDV** | **M4** | **STDV** | **(%)** | |
| **Group A** | **12.0** | 1.0 | | | | | **6.7** | 1.1 | **4.6** | 0.6 | **62%** | **Y** |
| **Group B** | **12.3** | 0.6 | | | | | **11.3** | 1.5 | **9.3** | 2.1 | **24%** | **N** |
| **Group C** | **12.6** | 0.6 | **12.3** | 1.1 | **11.6** | 1.5 | **10.0** | 1.0 | **9.0** | 1.7 | **29%** | **N** |
| **Group D** | **11.3** | 0.6 | **11.3** | 1.5 | **11.0** | 0.0 | **9.7** | 2.1 | **9.3** | 1.1 | **18%** | **N** |
| **Group E** | **11.6** | 1.1 | **11.6** | 1.1 | **11.3** | 0.6 | **9.3** | 0.6 | **8.0** | 1.0 | **31%** | **N** |
| **Group F** | **12.3** | 1.1 | **11.6** | 1.1 | **10.6** | 0.6 | **6.0** | 1.0 | **3.6** | 0.6 | **71%** | **N** |

| | | | | | | | | | | | | |
|---|---|---|---|---|---|---|---|---|---|---|---|---|
| (M=month): | | | | | | | | | | | | |

scores consist of subjective sleep quality, sleep latency (i.e., how long it takes to fall asleep), sleep duration, habitual sleep efficiency (i.e., the percentage of time in bed that one is asleep), sleep disturbances, use of sleeping medication, and daytime dysfunction.

Each item is weighted on a 0-3 interval scale. The global PSQI score was calculated by totaling the seven component scores, providing an overall score ranging from 0 to 21, where lower scores denote a healthier sleep quality. A higher number indicates more sleep-related concerns. A score of 5 or greater indicates a "poor sleeper". The results are presented in table 2, herein below.

**Table 2**

| **Daily Dosage** | **CBD (mg)** | **EPA+ DHA (mg)** | **DHA (mg)** | **EPA (mg)** | **Polar-EPA (mg)** |
|---|---|---|---|---|---|
| **Group A** | 200 (High) | | | | |
| **Group B** | 50 (Low) | | | | |
| **Group C** | 50 (Low) | 300 (EPA) | | | |
| | | 200 (DHA) | | | |
| **Group D** | 50 (Low) | | 500 | | |
| **Group E** | 50 (Low) | | | 500 | |
| **Group F** | 50 (Low) | | | | 500 |

Omega-3 supplements, on a stand-alone basis, show a mild improvement in sleep quality. High-dose CBD was more efficient that low-dose CBD in improving PSQI score, but had a sedation effect. Results show a great difference in the combined effect between various Omega-3s and low-dosage CBD. While all Omega-3 oils + low-dose CBD showed higher efficacy compared with low-dose CBD on a stand-alone basis, only Nannochloropsis oil, which contains polar-lipid (glycolipid and phospholipid) EPA, was found to create a synergetic effect. The PSQI improvement after 2 months was about 3X higher than administrating low-dose CBD only and over 2X higher than fish oil+CBD. The Nannochloropsis oil +low dose CBD created a similar PSQI improvement as high-dose CBD, but without the sedation effect. On the other hand, DHA Omega-3, from Schizochytrium Oil, did not create any effect at all. Group F, months 1-2, indicate the improvement of PSQI score with Nannochloropsis (EPA only) on a stand-alone basis (~15%). Group B, months 3-4, indicate the improvement of PSQI score with low-dose CBD on a stand-alone basis (~18%). Group F, months 3-4, indicate the synergistic effect (64% improvement), indicating that the improvement is not a mere aggregation of features, but a product of a synergistic effect. This is the only group were the PSQI score fell under the "poor sleep" threshold (<5).

## Claims

1. A composition comprising: Nannochloropsis oil and Cannabidiol "CBD".

2. The composition according to claim 1, wherein said Nannochloropsis oil forms at least 20%wt of the composition and/or said CBD forms at least 10%wt of the composition.

3. The composition according to claim 1 or 2, wherein the ratio between said Nannochloropsis oil and CBD is 1:1.

4. The composition according to claim 1 or 2, wherein the amount of Nannochloropsis oil is 500 mg and the amount of CBD is 50 mg.

5. The composition according to claim 1 or 2, wherein the Nannochloropsis oil comprises glycolipid and phospholipid Eicosapentaenoic acid "EPA".

6. The composition according to any one of the preceding claims being in the form of an oily solution, an oil-in-water solution and/or a dry powder.

7. A composition according to any of claims 1-6, for use as a medicine.

8. A composition according to any of claims 1-6, for use in the treatment of a disease or disorder selected from sleep disorder, insomnia and any combinations thereof.

## Patentansprüche

1. Zusammensetzung, umfassend: Nannochloropsis-Öl und Cannabidiol "CBD".

2. Zusammensetzung nach Anspruch 1, wobei das Nannochloropsis-Öl mindestens 20 Gew.-% der Zusammensetzung ausmacht und/oder das CBD mindestens 10 Gew.-% der Zusammensetzung ausmacht.

3. Zusammensetzung nach Anspruch 1 oder 2, wobei das Verhältnis zwischen dem Nannochloropsis-Öl und CBD 1:1 beträgt.

4. Zusammensetzung nach Anspruch 1 oder 2, wobei die Menge an Nannochloropsis-Öl 500 mg und die Menge an CBD 50 mg beträgt.

5. Zusammensetzung nach Anspruch 1 oder 2, wobei das Nannochloropsis-Öl Glykolipid und Phospholipid Eicosapentaensäure "EPA" umfasst.

6. Zusammensetzung nach einem der vorhergehenden Ansprüche, die in Form einer öligen Lösung, einer Öl-in-Wasser-Lösung und/oder eines Trockenpulvers vorliegt.

7. Zusammensetzung nach einem der Ansprüche 1 bis 6 zur Verwendung als Arzneimittel.

8. Zusammensetzung nach einem der Ansprüche 1 bis 6 zur Verwendung bei der Behandlung einer Krankheit oder Störung, ausgewählt aus Schlafstörungen, Schlaflosigkeit und beliebigen Kombinationen davon.

## Revendications

1. Composition comprenant : de l'huile de Nannochloropsis et du cannabidiol, « CBD ».

2. Composition selon la revendication 1, dans laquelle ladite huile de Nannochloropsis représente au moins 20 % en poids de la composition et/ou ledit CBD représente au moins 10 % en poids de la composition.

3. Composition selon la revendication 1 ou 2, dans laquelle le rapport entre ladite huile de Nannochloropsis et ledit CBD est de 1:1.

4. Composition selon la revendication 1 ou 2, dans laquelle la quantité d'huile de Nannochloropsis est de 500 mg et la quantité de CBD est de 50 mg.

5. Composition selon la revendication 1 ou 2, dans laquelle l'huile de Nannochloropsis comprend des glycolipides, des phospholipides, et de l'acide eicosapentaénoïque, « EPA ».

6. Composition selon l'une quelconque des revendications précédentes, se présentant sous la forme d'une solution huileuse, d'une solution d'huile dans l'eau et/ou d'une poudre sèche.

7. Composition selon l'une quelconque des revendications 1 à 6, pour une utilisation en tant que médicament.

8. Composition selon l'une quelconque des revendications 1 à 6, pour une utilisation dans le traitement d'une maladie ou d'un trouble choisi parmi les troubles du sommeil, l'insomnie, et toute combinaison de ceux-ci.
